Europäisches Patentamt

European Patent Office

Office européen des brevets

(11) Publication number: **0 105 364**
**B1**

(12) **EUROPEAN PATENT SPECIFICATION**

(45) Date of publication of patent specification: **28.03.90**

(51) Int. Cl.⁵: **A 61 K 37/00**

(21) Application number: **83901853.8**

(22) Date of filing: **14.04.83**

(86) International application number:
**PCT/US83/00543**

(87) International publication number:
**WO 83/03546 27.10.83 Gazette 83/25**

(54) **TEMPERATURE SENSITIVE REASSORTANT VIRUSES AND A VACCINE AGAINST EQUINE INFLUENZA.**

(30) Priority: **16.04.82 US 369319**

(43) Date of publication of application:
**18.04.84 Bulletin 84/16**

(45) Publication of the grant of the patent:
**28.03.90 Bulletin 90/13**

(84) Designated Contracting States:
**AT BE CH DE FR GB LI LU NL SE**

(56) References cited:
**GB-A-1 067 463**
**US-A-3 518 347**
**US-A-3 992 522**

**Science, vol. 160, 1968, p. 74-76 E.D. Kilbourne:
"Recombination of influenza A viruses of human
and animal origin."**

**Bulletin of the world health organisation, vol.
41, 1969, p. 447-452. J.A. Kasel et
al.:"Experimental infection in man and horses
with influenza A viruses."**

(73) Proprietor: **CORNELL RESEARCH
FOUNDATION, INC.**
**East Hill Plaza**
**Ithaca, N.Y. 14850 (US)**

(72) Inventor: **COGGINS, Leroy**
**309 Kelso Court**
**Cary, NC 27511 (US)**
Inventor: **MURPHY, Brian R.**
**5410 Tuscarawas Road**
**Glen Echo, MD 20816 (US)**
Inventor: **HOLMES, Dorothy F.**
**181 Cayuga Street**
**Groton, NY 13073 (US)**
Inventor: **ANGUISH, Lynne J.**
**200 Lower Creek Road**
**Ithaca, NY 14853 (US)**
Inventor: **GILLESPIE, James H.**
**45 Sunnyslope Road**
**Ithaca, NY 14853 (US)**

(74) Representative: **Kraus, Walter, Dr. et al**
**Patentanwälte Kraus, Weisert & Partner**
**Thomas-Wimmer-Ring 15**
**D-8000 München 22 (DE)**

Courier Press, Leamington Spa, England.

**(56)** References cited:

Biological Abstracts, vol. 62, no. 7, October 1, 1976, page 3656, ref. no. 36966; Philadelphia, US. R.Ya. Podchernyaeva et al.: "New data on the recombination of orthomyxoviruses."

Chemical Abstracts, vol. 95, no. 1, July 6, 1981, page 336, ref. no. 3288x; Columbus, Ohio, US. Yu. Z. Gendon et al.: "Analysis of genome composition and reactogenicity of recombinents of cold-adapted and virulent virus strains."

American Journal of Veterinary Research, vol. 43, no. 5, issued May 1982 L.J. Brundage-Anguish et al., "Live Temperature-Sensitive Equine Influenza Virus Vaccine: Generation of the Virus and Efficacy in Hamsters", see pages 869-874

Virology, Vol. 81, no. 1, issued August 1977, C. Scholtissekl et al., "Correlation of Pathogenicity and Gene Constellation of an Influenza A Virus (Fowl Plague)", see pages 74-80

Virology, vol. 112, no. 2, issued 30 July 1981 M.D. Tolpin et al., "Genetic Factors Associated with Loss of the Temperature-Sensitive Phenotype of the Influenza A/Alaska/77-ts-1A2 Recombinant during Growth in Vivo", see pages 505-517

Journal of the Royal Society of Medicine, vol. 75, issued October 1982 D.J. Alexander, "Ecological Aspects of Influenza A Viruses in Animals and Their Relationship to Human Influenza: a Review", see pages 799-811

Acta Veterinaria Academiae Scientarum Hungaricae, vol. 24, no. 3, issued 1974, J.Romvary et al., "Influenza Infection in Horse Stocks Caused by Equine A-1 and Hong Kong Subtype in Hungary", see pages 457-461

Bulletin of the world health organisation, vol. 34, issued 1966 N.Masurel et al., "Studies on the Content of Antibodies for Equine Influenza Viruses in Human Sera", see pages 885-893

## Description

This invention relates to a temperature-sensitive virus capable of providing an equine influenza vaccine.

U.S. Patent No. 3,992,522 teaches temperature-sensitive reassortant mutant viruses, methods for their production and vaccines derived therefrom. One could not predict from this art, however, that a reassortant virus formed from a human influenza virus and an equine influenza virus would provide a reassortant virus s which could be used to provide a vaccine which on the one hand would not cause serious disease in an equine, yet on the other hand would induce effective resistance in an equine to equine influenzal disease produced by challenge with a virulent wild-type equine influenza virus. Temperature-sensitive (ts) mutation affords the possibility of site-specific attenuation for the lower respiratory tract. The acquisition of temperature-mutant defects in a strain of influenza virus has been shown in mice to be associated with diminished virulence, though the antibody producing stimulus remained (British Medical Journal, 1969, 3:757—758). Replication of mutants with markedly restricted growth at 37°C—38°C appears to be greatly limited in the lower respiratory tract, the major site of significant pathology, which has a temperature of 37°C in humans. However, the mutants should grow with reasonable efficiency in the cooler passages of the upper respiratory tract, which has a temperature of 32°—34°C. In this manner, ts mutants grow primarily in the upper respiratory tract and stimulate immunologic defense mechanisms without producing symptoms in the lower tract. Additionally, ts mutants are often partially defective at permissive temperatures (32°—34°C), and this property offers the possibility of attenuation for the upper respiratory tract as well.

Much of the prior art relative to the present invention is of the literature variety, e.g.:

Murphy et al., "Temperature-Sensitive Mutants of Influenza Virus II Attenuation of ts Recombinants for Man", *The Journal of Infectious Diseases,* Vol. 127, No. 2, August 1972, pages 170—178.

Murphy et al., "Temperature-Sensitive Mutants of Influenza Virus III Further Characterization of the ts-1 [E] Influenza A Recombinant (H3N2) Virus in Man", *J. of Infectious Diseases, 128:* 478—487, 1973.

Beare et al., "Recombinant Influenza-A Viruses as Live Vaccines for Man", *Lancet 2:* 1271—1273, 1971. This Beare journal article does refer, as the title indicates, to a recombinant influenza-A virus as live vaccines for man; however, at page 1272, Table 2, it shows that there is no correlation between restricted growth at 39° and attenuation. This is brought out by the notation that the virulent parent virus (939) is restricted to 39° and the attenuated virus (PR8) replicates well as 39°. Additionally, clone 7 grows well at high temperature and is virulent, whereas clone 64C similarly grows well at 39° but is attenuated. Beare demonstrates simply that the mating of an avirulent virus (PR8) and a virulent virus (939) can give rise to clones of viruses with a spectrum of virulence for man. However, in this article there is no specific characteristic; i.e., temperature sensitivity, associated with this attenuation. The Beare article also utilizes an avirulent virus produced by serial passage in animal and tissue culture rather than by chemical mutagenesis.

Maassab et al., "Hybrid Formation of Influenze Virus at 25°C",, *Fed. Proc., 30:* 413, 1971 [abstract]. This immunology abstract and the parent article, *Proc. Soc. Exp. Biol. and Med., 139:* 768, March 1972, relate to the procedure of Dr. Maassab to produce attenuated viruses of influenze A virus, which is similar to but distinct from the present procedure. Maassab produces an attenuated parent virus by growth of virus at low temperature, a well-known technique for the production of live attenuated virus vaccine strains. Live measles vaccine have been produced in this manner. The present chemical technique involves the chemical mutagenesis of influenze A virus and the subsequent isolation of viruses that are temperature sensitive. The end result is to produce viruses that grow at 34° but not at 39°, and these are the ts attenuated viruses. The Maassab technique, which involves a transfer of a defect from an attenuated strain to a new wild-type virus by genetic recombination, is similar to the present invention, but the method of production of attenuated viruses is different.

MacKenzie, "Virulence of Temperature-Sensitive Mutants of Influenza Virus", *Br. Med. J., 3:* 757—758, 1969. With reference to this MacKenzie article, it is noted that distinct from the present development, MacKenzie did not work with recombinant ts viruses, although he did demonstrate the ts mutants of influenza A viruses produced by chemical mutagenesis were attenuated for animals. More important, the virus preparations in the journal article were not suitable for human use and no attempt was made to pass these ts defects to different influenza A viruses by recombination.

Recent epidemiological studies (Ingram et al., *J. Eq. Med. and Surg.,* Suppl. *1:* 329—338, 1978; Kemen, *Proc. Am. Assoc. Equine Practitioners, 20:* 119—126, 1974; and Kemen, *Hoofbeats,* January 1976, pp. 62—63), undertaken in areas of high equine density such as racetracks, training areas, shows and breeding farms have shown that the most important agent causing clinical respiratory disease in the horse is the equine influenza virus. Presently available inactivated equine influenze vaccines have several limitations. First, because of occasional deleterious side-effects in vaccinated horses and incomplete protection, many horsemen are abstaining from a regular vaccination program for their animals. Second, these vaccines provide only a short-lived program which lasts only 3—4 months under intense challenge and requires several vaccinations each year to provide adequate protection (Ingram et al., *supra;* Burrows, *Amer. Assoc. Equine Pract.,* 1979, p. 37—48).

Unlike the human influenza viruses, there has not been significant antigenic drift in the two equine influenza viruses (Burrows, *supra*). In addition, infection of horses with the wild-type virus provides a high degree of protection as indicated by the high frequency of disease in two- and three-year-olds with little clinical evidence of reinfection in previously exposed older horses (Ingram et al., *supra;* Kemen, *supra*). These factors support the idea that the development of an effective live virus vaccine for equine influenza would be valuable in the control of this disease since a live vaccine might stimulate immunity like that of natural infection.

In recent years, conditional-lethal, temperature-sensitive mutants of human influenza A viruses have been produced, characterized and evaluated as candidate live virus influenza vaccines in humans with some promising results (Chanock and Murphy, *Rev. Infect. Dis., 2:* 421—432, 1980). The use of ts mutants as vaccines offers several advantages. The location of the ts lesion on the viral genome can be determined and the level of *in vitro* temperature sensitivity of plaque formation measured in the laboratory (Murphy et al., *J. Infect. Dis., 130:* 144—149, 1974; Spring et al., *Virology, 66*: 542—550, 1975; and Murphy et al., *Infect. Immun., 20:* 665—670, 1978).

Attenuated ts vaccine viruses can be evaluated for genetic stability during production, experimental trials, and later usage in the field with the ts lesion serving as a marker for vaccine virus.

Ts reassortant clones which are restricted in replication *in vitro* at 37—38°C multiply efficiently in the upper respiratory tract of man and hamsters and induce local and systemic immune responses which protect against wild-type influenza virus (Murphy et al., *Infect. Dis., 126*: 170—178; Murphy et al., *J. Infect. Dis., 128*: 479—487, 1973; Murphy et al., *Virology, 88*: 244—251, 1978; Jennings et al., *Fed. Proc., 37*: 2072—2073, 1978, Mills et al., *J. Infect. Dis., 123*: 145—157, 1971; and Richman et al., *J. Infect. Dis., 134*: 585—594, 1976). However, because the replication of the virus is sensitive to temperature, these viruses replicate inefficiently in the warmer, lower respiratory tract (approximately 38°C in the horse) and, therefore, should not produce the cough characteristic of the wild-type equine influenza virus infection. It is hoped that the increased stimulation of local and systemic immune systems by viral replication could decrease the need for frequent vaccinations, with perhaps annual vaccination in horses being sufficient (Chanock et al., *Viral Immunology and Immunopathology,* 1975, Academic Press, NY, P 291—316).

GB—A—1 067 463 relates to the preventive treatment of equine influenza which comprises inocculating the animal with a human influenza virus vaccine. This is an old technology which never proved effective. This patent does not unnoticely consider recombination of human equine genes to form an effective safe vaccine.

US—A—3 518 347 is directed to a mixture inactivated viruses, not recombinants, but simple mixtures. In no way this citation anticipates the recombinants especially when history has demonstrated that the technique has not worked well.

Kilbourne, Science 160:74—76, shows hybridized equine/human strains apparently useful for humans, not for horses. The reference speaks of divergent neuraminidase and hemagglutinin antigens, and is silent as to a temperature-sensitive gene. In Applicant's preferred embodiments the recombinants have both antigens from the equine virus. Clearly what Applicant has invented is not related to what the citation teaches.

Kassel et al., Bull. W. H. O. 41:447—452, shows that there is some cross infection between certain human $A_2$ viruses and horse $A_2$ viruses. But in horses "no heterologous antibody responses to A/Equie-Z viruses were detected". Nothing in these data leads one toward the vaccine of the present invention.

Podchernyaeva et al., Biological Abstracts 62:36966 (1976), deals with mammalian (e.g. horse)-avian influenza recombinants and is clearly not teaching as to horse-human recombinants.

Gendon et al., C. A. 95:3288x, does not show any cross species recombination and only shows introduction of (ts) gene in a human recombinant leads to non-reactogenicity.

US—A—3 992 522 deals exclusively with the development of an influenza vaccine for man. The present equine vaccine is exclusively for horses and ponies and has been proven to be safe and non-infectious to humans handling it. The fact that human/human recombinants are possible effective vaccines is not predictive that certain horse/human recombinants would be both safe and effective as a vaccine for horses.

This invention relates to a temperature-sensitive virus capable of providing an equine influenza vaccine which is a reassortant mutant virus, formed by mating a wild-type equine influenza virus of the $A_1$ or $A_2$ subgroups with chemically mutagenized and temperature-sensitive human influenza virus having a shut off temperature in the area of 37°C to 39°C, said reassortant virus having at least one temperature-sensitive gene and at least two but no more than six human influenza virus derived genes, and being further characterized as having the serotype of the equine influenza virus and the temperature-sensitive defect of the human virus.

In general, the present product is obtained according to a method for producing a temperature-sensitive hybrid human/equine influenza-type virus which comprises mating a chemically mutagenized temperature-sensitive human influenza virus having at least one and preferably at least two ts lesions, with a virulent wild-type equine influenza virus to produce a new reassortant (sometimes called recombinant) virus with equine anti-genic components. The new reassortant viruses of the invention have at least two but no more than six of its eight genes derived from the human ts mutant with the remaining genes being derived from the wild-type equine virus.

As to the temperature-sensitive human influenza virus which can be employed to form the reassortant viruses of the invention, virtually any chemically mutagenized conditional-lethal temperature-sensitive (ts) mutants containing at least one and preferably at least two (ts) mutants can be employed. U.S. Patent No. 3,992,522, hereby incorporated by reference in its entirety, describes the preparation of various of such ts mutants from human influenze A type viruses including:

Influenza A/1965-ts-1 (H2N2)
Influenza A/Hong Kong/1968-ts-1[A] (H3N2)
Influenza A Double recombinant 10B (H0N2)
Influenza A/Hong Kong/1968-ts-1[E] (H3N2)

as well as further recombinant viruses such as one denoted ts-1[E] which was produced from wild-type influenza A/Hong Kong/1968 (H3N2) and the 1965-ts-1 mutant noted above.

As to the wild-type equine influenza virus employed to form the reassortant viruses of the invention any virulent equine influenza virus of either the $A_1$ or $A_2$ subgroups may be employed.

The generalized method of production of temperature-sensitive recombinants involves a mixed infection of cells grown in tissue culture incubated at a permissive temperature (34°C) using two viruses: (1) a temperature-sensitive virus of one serotype, and (2) another serotype non-ts virus that one desires to attenuate. Antiserum to the ts virus must be available that will neutralize the ts parent virus but have no effect on the non-ts virus. The cells are infected simultaneously at a multiplicity of infection of 1 for each parent virus. The progeny of this mating is then plaqued on monolayer cultures at the permissive temperature in the presence of antisera to the ts parent virus. The virus present in the plaques is then inoculated into tissue culture, incubated at the permissive temperature, and harvested. The virus present in this harvest is then characterized for its serotype and temperature sensitivity. The virus which has both the serotype of the non-ts parent and the ts defect of the ts parent is considered to be the desired recombinant. The desired recombinant ts virus is then subjected to two successive plaque-to-plaque passages.

After the last plaque passage, the virus is then grown up in tissue culture and the serotype and ts characteristic again determined. This method of plaque-to-plaque purification insures that the recombinant virus is both genetically homogeneous and stable. The ts recombinant virus is ready at this point to be used as a seed for production of larger quantities of virus.

## Example

Temperature-sensitive (ts) reassortants of an equine influenza, subtype A-1, were produced by mating a human influenza ts donor virus with an equine influenza A/Cornell/16/74 wild-type (wt) virus and by isolating a ts reassortant virus possessing the equine hemagglutinin and neuraminidase surface antigens. Two equine ts reassortant clones, 8B1 and 71A1, were produced which has an *in vitro* shutoff temperature for plaque formation of 38 and 37°C, respectively. The human ts donor virus had ts mutation(s) on the polymerase 3 (P3) and nucleoprotein (NP) genes so that a ts equine reassortant virus could have either or both of these ts genes. It was found by complementation analysis that reassortant clone 8B1 had a ts lesion on the P3 gene and clone 71A1 had ts lesions on the NP and P3 genes. An analysis of the parental origin of the genes in each ts equine reassortant virus indicated that clone 8B1 received 6 of its 8 genes and clone 71A1 three of its 8 from the equine parent virus, the remainder genes being from the human ts donor virus. The growth of both clones was restricted in the lungs of hamsters, but similar to that of the equine wild-type virus in the nasal turbinates. Each virus isolate obtained from the hamster's lungs or nasal turbinates retained the ts phenotype. These findings form the basis for further evaluation of the equine ts reassortant viruses for their level of attenuation and immunogenicity in horses.

## MATERIALS AND METHODS
### Viruses

The wild-type equine influenza virus, subtype A-1, A/Cornell/16/74 Heq1 Neq1 [H7N7 by new WHO nomenclature (Bulletin of World Health Organization *58*: 585—591 (1980)] was originally isolated in 1973 from an outbreak of equine influenza in Florida. It was subsequently passaged intranasally through 4 Shetland-type ponies to check for virulence, then grown in Madin-Darby Canine Kidney Cells (MDCK), plaque purified twice by passage at 34°C and 39°C, and then grown twice in MDCK culture (TCID $10^{6.5}$/ml). This virus was designated A/Cornell/74 clone 2A. Before being used in the production of a temperature-sensitive reassortant equine influenza, clone 2A was shown to produce a fever, hyperemia and congestion of the nasal mucosa, and a serious nasal discharge in experimental ponies. Wild-type human A/Udorn/307/72 (clone 3A1) (H3N2), which had been passaged 6 times in primary calf kidney cells BK, once in eggs and one in MDCK cells, was used as a control virus in the hamster studies and tissue culture assays.

The production of the human influenza A ts mutant used as a donor of its ts genes to the A/Cornell/74 equine virus has not been described previously. This ts virus contains ts mutations on the genes coding for the P3 polymerase protein and the nucleoprotein (NP) gene and was produced by mating the A/Victoria/75-ts-1A2 (clone 65A1) $H3_{75}N2_{65\ or\ 68}$ virus (ts P3 gene) (Murphy, B. R., Markoff, L. J., unpublished observation) with the A/Udorn/307/72-ts-368 $H3_{72}N2_{72}$ virus (ts NP gene). This latter ts virus was produced by chemical mutagenesis by Dr. Lewis Markoff at the Laboratory of Infectious Diseases, National Institutes of Health, Bethesda, Maryland and was shown by complementation and segregational analysis to contain a ts NP gene (Dr. Markoff, unpublished observation). A reassortant virus from the above mating, designated A/Udorn/72-ts-20A1 $(H3_{72}N2_{72})$, was shown by genetic analysis to contain the ts NP gene from the A/Udorn/72 ts 368 virus using methods previously described (Murphy et al., *Infect. Immun., 20*: 665—570 1978; Spring et al., *Virology, 61*: 512—532 (1975); Murphy et al., *Infect. Immun., 20*: 671—677, 1978; Massicot et al., *Virology, 101*: 242—249, 1980). The A/Udorn/72-ts-20A1 reassortant was passaged five times in BK including a plaque-to-plaque passage (titer $10^{6.5}TCID_{50}/ml$) and had a shutoff temperature for plaque formation of 37°C. The two ts parents of clone 20A1, A/Udorn/72 (clone 368A2) and A/Victoria/75-ts-1A2 (clone 65A1), were used in complementation studies as "probe" viruses. Clone 368A2 had been passaged four times in Bk and twice in eggs. It contained a ts lesion on the NP gene (complementation group 2). Clone 65A1 has been passaged 5 times in Bk and once in eggs and had a ts lesion on the P3 gene (complementation group 2) (Spring et al., *Virology, 66*: 512—532, 1975; Mills et al., *J. Infect. Dis., 123*: 145—157, 1971).

*Tissue Culture and Infectivity Assays*

Infectivity and plaque assays have been described previously (Murphy et al., *Infect. Immun., 20*: 665—670, 1978) except that 12-well plastic tissue culture plates were used for plaque tests instead of 6-well plates. The MDCK cell monolayers used throughout this study (Tobita, et al., *Med. Microbiol. Immunol. 162*: 9—14 (1975); Nath et al., *Am. J. Vet. Res., 38*: 1059—1061, 1977) were prepared by Flow Laboratories. Media consisted of either Eagles minimal essential medium with Earle's salts or 50% Eagles #2 and 50% Medium 199 (M A Bioproducts) containing 0.29 mg L-glutamine, 2.5 μg amphotericin B and 66 μg genatmycin per ml. For cell growth, 10% fetal bovine serum (screened for suitability for replication of influenza virus) was included; for virus growth medium, serum was deleted and trypsin was added at a final concentration of 1 μg/ml. In the hamster infectivity studies, transport medium consisting of 50% Eagles #2 and 50% Medium 199 and containing 0.5% gelatin and twice the concentration of antibiotics was used to collect the virus from tissues. Plaque overlay consisted of L-15 medium with L-glutamine, trypsin, and antibiotics at the above concentrations plus 0.8% agarose. The addition of trypsin to virus growth medium enhanced the formation of visible plaques which made addition of erythrocytes to visualize titration endpoints unnecessary in most cases (Appelyard et al., *J. Gen. Virol., 25*: 351—357, 1974; Tobita, et al., *Med. Microbiol. Immunol., 162*: 9—14, 1975; Klenk et al., *Virology, 101*: 242—249, 1980).

*Production of Equine Reassortants* :

The procedures for the production of reassortants were similar to those previously described (Murphy et al., *Virology, 66*: 533—541, 1975; Spring et al., *Virol., 66*: 512—532, 1975). Briefly, A/Udorn/72 ts-20A1 and A/Cornell/74 clone 2A were mixed at an M01 of 1 for each virus and then added to washed monolayers in 6-well plastic tissue culture plates. After a 1 hour adsorption at room temperature, the cells were washed 5 times, overlayed with plaqueing medium and incubated for 24 hours at 34°C. The progeny viruses were harvested, serially diluted (10-fold) and incubated for 1 hour at room temperature with an equal volume of a 1:200 dilution of hyperimmune ferret antiserum against A/England/42/72 (H3N2) virus. This concentration of antiserum had previously been shown to reduce the titer of the A/Udorn/72-ts-20A1 virus by at least 100-fold but did not neutralize the equine parent virus. The mixtures were then inoculated onto mono-layers in 6-well plates and observed for plaque formation. A/Cornell/74-ts reassortant viruses were selected from the reassortant progeny and cloned as previously described (Richman et al., *Virology, 66*: 551—562, 1975). The virus clones were plaque purified 3 times.

*Characterization of Equine ts Reassortants*

The procedure for determining the efficiency of plaque formation of selected virus clones at 34, 36, 37, 38 and 39°C has been described (Murphy et al., *Infect. Immun., 20*: 665—670, 1978; Murphy et al., *Infect. Immun., 20*: 671—677, 1978), except that 12-well plates were used in the present study.

Plate complementation tests, using A/Victoria/75-ts-65A1 and A/Udorn/72-ts-368A2 viruses as ts probes, were performed as previously described (Murphy et al., *Infect. Immun., 20*: 665—670, 1978; Spring et al., *supra*; Murphy et al., *Infect. Immun., 20*: 671—677, 1978), except that the viruses were not first incubated at 4°C.

The hemagglutinin present on each ts reassortant was characterized by standard microtiter hemagglutination inhibition (HI) tests using monospecific goat anti-H equi-1 and ferret anti-H3 antisera. The neuraminidase present on the reassortant was characterized using neuraminidase inhibition (NI) tests and goat anti-N equi-1 antiserum as previously described (Murphy et al., *New Engl. J. Med., 286*: 1329—1332, 1972; Fedson et al., *J. Immunol., 107*: 730—737, 1971).

*Polyacrylamide Gel Electrophoresis (PAGE) of Equine ts Recombinant Virion RNA*

To determine the parental origin of individual genes in the equine ts reassortant clones 71A1 and 8B1, virion RNA (v RNA) from these two viruses was analyzed as previously described (Massicot et al., *Virology, 101*: 242—249, 1980). The migration patterns of v RNA obtained from reassortant viruses were compared to that of the A/Udorn/72-ts-20A1 and Cornell 74 wild-type clone 2A parent viruses.

The conditions used to see differences in the migration rates of the parental RNAs were as follows: genes, 2, 3, 4, and 8 on a 20 cm gel of 2.8% acrylamide and 6M urea for 17.5 hours at 28.5°C and 95 volts; gene 1 on a 20 cm gel of 3.0% acrylamide and 6M urea for 19 hours at 29°C and 100 volts; and genes 5 and 7 on a 20 cm gel of 2.6% acrylamide run in 6M urea for 16 hours at 26°C and 110 volts. Gene 6 (nucleoprotein) was kindly identified by Kathleen Van Wyke at St. Jude's Hospital using monoclonal antibodies in an enzyme-linked immunosorbent assay as previously described (Van Wyke et al., *J. Virol., 35:* 24—30, 1980).

*Hamster Studies*

After intraperitoneal pentobarbitol anesthesia, groups of six-week-old female Golden Syrian hamsters (Lakeview Animal Farms, Newfield, New Jersey), were inoculated intranasally with 0.1 ml of virus suspension that contained $10^{5.5}$TCID$_{50}$. Five to 8 animals were sacrificed daily for 4 days (clones 2A, 8B1, 71A1 and 20A1) or 2 days (clone 3A1) and lungs and nasal turbinates were removed aseptically. The lungs were ground in 7 ml of transport medium in a Ten-Broeck type tissue grinder (10% w/v); turbinates were ground with an apothecary mortar and pestle with 0.5 ml of transport medium and roughly 3 g of sterile sea sand, after which 5.0 ml of medium were added (5% w/v). All samples were then centrifuged and the supernatant divided into 3 aliquots and stored at −70°C until titrated. Virus was titrated in 24-well disposable tissue culture plates at 34°C and the endpiont after 4 days of incubation was expressed as TCID$_{50}$/gm of tissue. The supernatants from the wells inoculated with the two lowest dilutions which showed viral CPE were harvested and tested for efficiency of plaque formation at 34 and 39°C to determine if there was loss of the ts phenotype after replication *in vivo*.

*Selection and Characterization of Cornell/74-ts Reassortant Viruses*

The reassortant progeny from the double infection of the human ts and equine wild-type viruses were characterized. The neuraminidase and hemagglutinin subtype, efficiency of plaque formation (EOP), shutoff temperature for growth and complementation group(s) of the parent strains and selected ts equine reassortant clones are shown in Table 1.

TABLE 1.  Characteristics of Parent Strains and A/Cornell/74-ts Recombinants

| Influenza A Virus | Hemagglutinin Subtype | Neuraminidase Subtype | Log-10 Reduction in Virus Titer (PFU/ 0.1 ml)* | | | | Shut-off Temperature[†] | Complementation [‡] Group of ts Lesion(s) |
|---|---|---|---|---|---|---|---|---|
| | | | 36° | 37° | 38° | 39° | | |
| A/Cornell/74 wild type | H-equi-1 | N-equi-1 | 0.1 | 0.1 | 0.1 | 0.1 | > 39 | NA |
| A/Udorn/72 ts clone 20A1 | $H3_{72}$ | $N2_{72}$ | 1.0 | > 3.4 | > 4.9 | > 4.9 | 37 | 1,2 |
| A/Cornell/74 ts recombinant clones | | | | | | | | |
| 8B1 | H-equi-1 | N-equi-1 | 0.3 | 0.6 | > 5.7 | > 5.7 | 38 | 1 |
| 71A1 | H-equi-1 | N-equi-1 | 0.9 | > 3.2 | > 5.4 | > 5.4 | 37 | 1,2 |

* Average of 2-5 tests.  Reduction at indicated temperature (°C) from titer at permissive temperature.

† Defined as the lowest temperature which gives 2 Log-10 reduction in titer.

‡ Complementation-recombination group 1 ts lesion is on the P3 gene, group 2 is on the NP gene.

NA = not applicable.

EP 0 105 364 B1

Of 77 viable plaques picked, two (2.6%) possessed the equine hemagglutinin and neuraminidase and the ts pheno-type (designated clones 8B1 and 71A1). Plaques were not produced at 39°C when clone 71A1 was grown in mixed culture with either ts virus probe in the complementation assay and this indicated that clone 71A1 had received both the ts NP and ts P3 gene from its A/Udorn/72-ts 20A1 parent. In addition, the clone 20A1 ts parent and its clone 71A1 equine ts progeny exhibited the same EOP (37°C shutoff temperature). When clone 8B1 was cultured with the ts probes, plaques were observed at 39°C in the 8B1 × 368A2 (NP lesion) mixed cultures but not in the 8B1 × 65A1 (P3 lesion) cultures. Thus, clone 8B1 possesses a ts lesion on the P3 but not on the NP gene and had a shutoff temperature of 38°C. An A/Cornell/74-ts reassortant that possessed only a ts NP gene was not isolated.

The parental origin of the genes in A/Cornell/74-ts clones 8B1 and 71A1 was determined by a comparison of the relative migration of RNA of parental and reassortant viruses in PAGE, Table 2. (These assignments of parental origin are based on antigenic analysis and on the order of migration of the v RNA segments fromt the human virus whose genetic map is known). The order of migration of the P1—3 genes of the equine virus were different from the Udorn. In the Udorn/72 preparation RNA 1, 2, and 3 are genes P3, P1 and P2, respectively, but in the Cornell/74 wild-type 1 preparation, RNA 1 is P2. The two RNA segments which migrated just ahead of the equi P2 gene must be the P1 and P3 genes of the equi virus but which RNA segment corresponds to which gene has not been determined. most of the genes of clone 71A1, including the ts, NP and P3 genes, migrated with the human Udorn/72-ts parent genes, indicating that they were derived from this parent. However, except for the P1 and ts P3 gene, all of the clone 8B1's genes co-migrated with the Cornell/16/74 clone 2A parent virus genes, indicating that clone 8B1's genes were derived mainly from its equine parent.

## TABLE 2. Genotype of Equine Influenza ts Reassortants

| Gene Coding For: | Clone 71A1 | Clone 8B1 |
|---|---|---|
| Polymerase 1 | Human | Human |
| Polymerase 2 | Human | Equine |
| Polymerase 3 | Human (ts) | Human (ts) |
| Hemagglutinin | Equine | Equine |
| Neuraminidase | Equine | Equine |
| Nucleoprotein | Human (ts) | Equine |
| Matrix proteins | Human | Equine |
| Nonstructural proteins | Equine | Equine |

*Replication of Parent and ts Progeny Viruses in Hamsters*

The replication of the A/Cornell/74-ts viruses was restricted in the lungs but not the nasal turbinates of hamsters. The $\log_{10}$ reduction in lung virus titer as compared to its wild-type virus was 5.7 for the human ts-20A1, 3.0 for ts-8B1 and 3.6 for ts-71A1. The frequency with which 8B1 was shed (Table 3) (% in animals) was similar to the wild-type 2A on all 4 days, starting with 100% of animals on day 1 and reducing to approximately 60% on day 4.

None of the hamsters which were infected with ts viruses shed ts+ (revertant) virus which indicates that the equine ts reassortant viruses, like their human ts parent, did not lose the ts phenotype in the hamster.

TABLE 3. Infection of Hamsters with Wild-Type and ts Viruses.

| Influenza A Virus | Animals Infected on Indicated Day* | | | | Genetic Stability # Isolates ts$^+$ / # tested** | |
|---|---|---|---|---|---|---|
| | 1 | 2 | 3 | 4 | Lungs | Nasal Turbinates |
| A/Cornell/74 wild type clone 2A | 100 | 89 | 75 | 80 | NA | NA |
| A Udorn/72 wild type clone 3A1 | 100 | 89 | ND | ND | NA | NA |
| A/Udorn/72 ts clone 20A1 | 89 | 75 | 63 | 25 | 0/2 | 0/19 |
| A/Cornell/74 ts recombinant clones | | | | | | |
| 8B1 | 100 | 100 | 75 | 43 | 0/12 | 0/25 |
| 71A1 | 63 | 63 | 38 | 14 | 0/2 | 0/14 |

* Infection defined as recovery of virus from the lungs and/or nasal turbinates.

** ts+ is defined as an isolate that produces plaques at the restrictive temperature (39°C). All viral isolates from lung and nasal turbinates were tested.

NA = Not applicable.

ND = Not determined.

EP 0 105 364 B1

One of the ts equine recombinants (8B1) was selected to begin immunogenicity and safety studies in horses. This virus was chosen because it grew to higher titer in the MDCK cell line and because it contained a higher % of genes derived from the equine parent and might, therefore, be expected to grow more readily in the horse. Ponies held in isolation units were screened for pre-existing antibody to Equin $A_1$ influenza virus, and were infected by aerosolization with 8B1 virus. Animals were observed daily for clinical signs. Temperatures were taken twice daily and naso-pharyngeal swabs for virus isolation were obtained for 7 days post-infection. 8B1 was found to successfully infect ponies as evidenced by virus recovery from the naso-pharynx and the subsequent development of serum hemagglutination-inhibting (HI) antibody in ponies. Clinical signs following infection with 8B1 were limited to slight nasal hyperemia and an occasional serious nasal discharge. None of the animals became febrile or developed a cough. Virus recovered from the nasal swabs was tested and determined to still retain its temperature-sensitive property.

Four weeks after 8B1 infection, ponies were challenged (also by aerosolization) with "wild type" equine influenza $A_1$ virus. The challange virus has been previously shown to induce fever and nasal discharge in inoculated horses and can be recovered from naso-pharyngeal swabs for up to 5 days. With one exception, the 8B1-vaccinated ponies had no febrile response following challenge and challenge virus was not recovered from the naso-pharynx.

Fourteen ponies were vaccinated with 8B1 and all were challenged with "wild-type" virus. Six additional ponies served as challenged controls.

TABLE 4. Development of a Temperature-Sensitive Equine Influenza Vaccine - Summary of Initial Experimental Ponies

| Pony No. | Vaccine doses | Pre-Inoculation Titers | Max. Post Vac. Titers | Max. Body Temp. post Vac. | Days to Maximum Titer | Post Vac. Virus Recovery (Days) | Max. Body Temp. Post Challenge | Post Chall. Wild-type Virus Recov. | Maximum Post Chall. Titer (HI) | Days to Maximum Titer |
|---|---|---|---|---|---|---|---|---|---|---|
| 600 | 1 | Neg. | 40 | 100.7 | 22 | 2,3,4,5,6 | 100.5 | None | 40 | 5 |
| 601 | 1 | Neg. | 10 | 100.6 | 29 | 2,3 | 104.1 | Days 2,3 | -- | - |
| 603 | 3 | Neg. | 20 | 100.8 | 14 | None | 100.0 | None | 40 | 14 |
| 605 | 3 | Neg. | 20-40 | 100.6 | 27 | None | 100.6 | None | 80 | 14 |
| 606 | 2 | Neg. | 10 | 100.7 | 27 | None | 99.9 | None | 40 | 14 |
| 617 | 2 | Neg. | 40-80 | 100.8 | 27 | None | 100.7 | None | 40 | 14 |
| 616 | 1 | Neg. | 10 | 100.9 | 21 | 3 | 100.8 | Day 3 | 20-40 | 21 |
| 625 | 1 | Neg. | 20 | 100.0 | 15 | 3 | 100.8 | None | 20 | 14 |
| 624 | 1 | Neg. | 10 | 100.6 | 21 | 3 | 100.9 | None | 20-40 | 14 |
| 622 | 1 | Neg. | 10 | 100.7 | 15 | 3,5 | 101.4 | None | 40 | 21 |
| 483F$_6$ | 1 | Neg. | 40 | 100.5 | 12 | 2 | 100.4 | None | 20 | 1 |
| 619 | 1 | Neg. | 20 | 100.6 | 28 | 2,3,4 | 100.2 | None | 20 | 1 |
| 621 | 1 | Neg. | 20 | 100.6 | 28 | 2,3,4 | 100.4 | None | 80 | 12 |
| 623 | 1 | Neg. | $\leq$ 10 | 100.8 | 7 | None | 101.2 | None | 10 | 26 |
| Challenge Controls | | | | | | | | | | |
| 515F$_6$ | - | Neg. | - | | - | | 104.0 | Days 2,3,4,5 | ND | - |
| 611 | - | Neg. | - | | - | | 102.2 | Days 2,3,4,5 | 80 | 21 |
| 618 | - | Neg. | - | | - | | 102.4 | Days 2,3,5 | 20 | 14 |
| 620 | - | Neg. | - | | - | | 101.4 | Days 2,3,5 | 20 | 14 |
| 451F$_6$ | - | Neg. | - | | - | | 102.0 | Days 2,3,4,5,6 | 80 | 14 |

EP 0 105 364 B1

## Claims

1. A temperature-sensitive virus capable of providing an equine influenza vaccine which is a reassortant mutant virus, formed by mating a wild-type equine influenza virus of the $A_1$ or $A_2$ subgroups with chemically mutagenized and temperature-sensitive human influenza virus having a shut off temperature in the area of 37°C to 39°C, said reassortant virus having at least one temperature-sensitive gene and at least two but no more than six human influenza virus derived genes, and being further characterized as having the serotype of the equine influenza virus and the temperature-sensitive defect of the human virus.

2. A virus as in claim 1 wherein the virus has six equine virus genes and two human virus genes.

3. A vaccine against equine influenza comprising the virus of claim 1.

4. A vaccine against equine influenza comprising the virus of claim 2.

## Patentansprüche

1. Temperatursensitives Virus, geeignet für die Herstellung eines Impfstoffs gegen Pferdeinfluenza, dadurch gekennzeichnet, daß est sich um ein reassortierendes, mutiertes Virus handelt, das durch Kreuzung eines Wildtypvirus für Pferdeinfluenza der Untergruppen $A_1$ oder $A_2$ mit einem chemisch mutierten und temperatursensitiven Humaninfluenzavirus mit einer Shut-off-Temperatur im Bereich von 37°C bis 39°C gebildet wird, wobei das reassortierende Virus mindestens ein temperatursensitives Gen und mindestens zwei, aber nicht mehr als sechs von dem Humaninfluenzavirus abgeleitete Gene besitzt und weiter durch den Serotyp des Pferdeinfluenzavirus und den Temperatursensitivdefekt des Humanvirus gekennzeichnet ist.

2. Virus nach Anspruch 1, dadurch gekennzeichnet, daß das Virus sechs Gene aus dem Pferdevirus und zwei Gene aus dem Humanvirus enthält.

3. Impfstoff gegen Pferdeinfluenza, dadurch gekennzeichnet, daß der das Virus nach Anspruch 1 enthält.

4. Impfstoff gegen Pferdeinfluenza, dadurch gekennzeichnet, daß der das Virus nach Anspruch 2 enthält.

## Revendications

1. Virus sensible à la température capable de fournir un vaccin contre la grippe équine, qui est un virus mutant de réassortiment, formé par appariement d'un virus de la grippe équine de type sauvage des sous-groupes $A_1$ ou $A_2$ avec un virus de la grippe humaine sensible à la température et ayant subi une mutagénèse chimique ayant une température d'arrêt dans la zone de 37°C à 39°C, ce virus de réassortiment étant caractérisé en ce qu'il a au mins un gène sensible à la température et au moins deux gènes mais pas plus de six gènes dérivés du virus de la grippe humaine et qu'il a de plus le sérotype du virus de la grippe équine et le défaut sensible à la température du virus humain.

2. Virus suivant la revendication 1, caractérisé en ce que le virus a six gènes du virus équin et deux gènes du virus humain.

3. Vaccin contre la grippe équine, caractérisé en ce qu'il comprend le virus suivant la revendication 1.

4. Vaccin contre la grippe équine, caractérisé en ce qu'il comprend le virus suivant la revendication 2.